# EUROPEAN PATENT APPLICATION

(11) **EP 3 181 101 A1**
(43) Date of publication of application: **21.06.2017**
(21) Application number: 15200804.1
(22) Date of filing: 17.12.2015
(51) Int. Cl.: A61F 5/445

(54) **OSTOMY BAG SYSTEM**

(71) Applicant: Dalina Medtech AB, 352 63 Växjö (SE)
(72) Inventor: FRIDLUND, Lina, 352 63 Växjö (SE); FRIDLUND, Dan, 242 62 Moheda (SE)
(74) Representative: Awapatent AB

(57) **Abstract**

A system and a method for emptying an ostomy bag (12) into a collecting bag (14) is disclosed. The ostomy bag (12) is connected to a waste collecting bag (14) by means of a first connector (C1) and a second connector (C2). By a single combined locking and opening maneuver, the user may lock the ostomy bag to the collecting bag such that they stay connected to each other during a subsequent emptying, and open valves in the two connectors (C1, C2) for providing a sealed flow path from said ostomy bag to said collecting bag. By a single unlocking and closing maneuver, the user may subsequently unlock the waste collecting bag (14) from the ostomy bag (12) and close both valves.

## Description

### TECHNICAL FIELD

The present inventive concept relates to the field of ostomy bags in general. More specifically, the inventive concept relates to an ostomy bag system for providing a secure and hygienic emptying of an ostomy bag using a waste collecting bag. The inventive concept also relates to a kit of connectors for use in such a system, a waste collecting bag for use in such a system, and a method for emptying an ostomy bag.

### BACKGROUND

An ostomy bag or pouching system includes a medical device, typically an ostomy bag, for collecting waste from a surgically diverted biological system (colon, ileum, bladder). The present invention may be used in connection with colostomies, ileostomies and urostomies. An ostomy bag is attached in an air- and water-tight manner over a stoma, allowing the stoma to drain into the ostomy bag. Such a system may also be referred to as an appliance, referring to a prosthesis as a mechanical replacement for a biological function. The attachment of the ostomy bag may be accomplished by e.g. a ring-shaped female connector arranged on a rear side of the ostomy bag and a ring-shaped male connector secured on the body of the person wearing the bag.

Known ostomy bags comprises open-end bags having a lower end or mouth that may be opened (e.g. by unfolding) to drain the contents of the bag into a toilet. Such an open end may be closed and secured by Velcro closures or clips.

Ostomy bags are normally emptied trough the openable mouth of the bag directly into a basin or into a toilet. This procedure involves a number of substantial problems, including unpleasant odor, risk of spilling during the emptying, risk of spreading diseases such as HIV, Hepatitis, etc for instance at hospitals, use of unclean toilets, rinsing problems after emptying involving splashing, hand contact with fecal residue, and a general problem of contamination of devises are areas around the area where the emptying is performed.

Some proposal has been made to use a disposable collecting bag for emptying the ostomy bag while the latter is still attached to the person wearing the bag.

US 2014/0371699 A1 (Feingold) discloses a closed ostomy bag system, wherein a discharge passage at the lower end of an ostomy bag extends into a receiving passage of a waste collecting bag to receive waste and flushing liquid from the ostomy bag. Slide fasteners connect the passages during the discharging operation. Nubs on the discharge passage engage registration openings in the receiving passage to register the slide fasteners with each other. A separate slide fastener extending across the receiving passage of the collecting bag is sealed after the ostomy bag has been clamped and prior to disengaging ostomy bag from the collecting bag for sanitary disposal. A cap is placed over the opening of the ostomy bag to contain any residue contaminants.

The system proposed in US 2014/0371699 A1 has a rather complicated construction and requires multiple maneuver steps by the use during the discharge operation. Also, the system presents the drawback that the waste collecting bag would present exposed unclean surfaces and parts after disconnection.

US 2010/217214 A1 (Hansen) discloses an arrangement for connecting fecal receiving bags together. The arrangement provides for a coupling arrangement establishing a fluid communication between the bags. This arrangement is also complicated to handle by the user and the overall problem of avoiding leakage and odor is not solved in a satisfactory manner.

US 4 387 713 discloses a unidirectional check valve for releasing waste from a primary ostomy bag into a secondary ostomy bag. The valve opens when the pressure in the primary ostomy bag becomes large enough. The person wearing the primary bag will also be wearing the check valve and the secondary bag, which is obviously not an attractive solution.

### SUMMARY OF THE INVENTION

According to a first aspect of the inventive concept, there is provided an ostomy bag system, comprising an ostomy bag for receiving bodily waste from an stoma, and a collecting bag for receiving bodily waste from the ostomy bag, wherein:
the ostomy bag is provided a first connector and the collecting bag is provided with a second connector, said first and second connectors being connectable to each other,
said first connector is provided with a waste outlet valve being moveable between a closed position and an open position,
said second connector is provided with a waste inlet valve being moveable between a closed position and an open position,
said first connector or said second connector is provided with at least one locking element, said locking element being moveable between an unlocked position in which the first and second connectors are not locked to each other and a locked position in which the first and second connectors are locked to each other, and
the system is arranged such that when said locking element is moved from its unlocked position to its locked position, said first and second valves are automatically brought into their open positions for defining a closed flow path from the ostomy bag to the collecting bag, and such that when the locking element is moved from its locked position to its unlocked position, said first and second valves are automatically brought into their closed positions for closing said flow path.

The inventive ostomy system provides a highly secure, closed system for emptying an ostomy bag by using a collecting bag which is temporarily connected and locked to the ostomy bag for receiving waste there from. The outlet valve of the ostomy bag and the inlet valve of the collecting bag would initially be in their closed positions. A user (which typically would be the person wearing the ostomy bag or as an alternative an assistant) will bring the first and the second connectors into engagement with each other. In this initial engaged position, the two connectors have contact with each other and are properly aligned with each other but are still not locked to each other and the two valves are still closed. Next, the user will bring the moveable locking element to its locked position, thereby locking the two engaged connectors to each other such that the two connectors are prevented from being disengaged from each other during emptying of waste from the ostomy bag into the collecting bag. As will be described below, this maneuver may be performed using a dedicated maneuver means, such as a finger grip on one of the connectors. According to the inventive concept, the system is arranged such that when the locking element is moved from its initial unlocked position to its locked position, the first and the second valves are automatically brought into their open positions for defining a flow path from the ostomy bag to the collecting bag via the two valves now being in their open positions. The waste can now be securely emptied from the ostomy bag through a closed and sealed flow path into the waste collecting bag.

After the emptying operation has been completed in the locked position, the user will move the locking element to its unlocked position, which in a preferred embodiment would involve a reverse movement. The system is arranged such that the two valves are then automatically brought into their closed positions, thereby closing the bags. The two connectors may then be disengaged from each other without any risk of leakage from the bags.

The inventive system offers a highly hygienic and secure way of emptying an ostomy bag into a collecting bag. The system will prevent any leakage and any odor. In preferred embodiments, the ostomy bag connector may be held completely uncontaminated on open surfaces thereof. Especially, the inventive system is designed as a fail-proof system since the valve opening and valve closing operation are operatively associated with the locking and unlocking operation. The valves are not being opened until the connectors are locked to each other, and the connectors cannot be disengaged from each other before the valves have been closed again.

The ostomy bag comprised in the inventive system would comprise at least one inlet opening arranged over a stoma for receiving waste there from, and a first outlet opening arranged at the outlet valve of the first connector. In one embodiment, the ostomy bag has no additional outlet openings and all emptying will be performed through the outlet valve and into the collecting bag. However, in order to allow the user to empty the collecting bag in a more conventional way as described above in connection with the prior art, for instance in situations where no waste collecting bag is available, embodiments of the inventive system may comprise an ostomy bag having an additional opening for emptying the bag. Such an additional opening may typically be designed as prior-art discharge openings at a lower end of the ostomy bag, for instance a mouth which may be opened and closed by unfolding and re-folding a part of the bag and sealed in a suitable way in its folded, closed position. Preferably, the inlet opening of the ostomy bag may be arranged at an upper end of the bag at a rear side of the bag facing the person wearing the bag, whereas the connector with the outlet valve is preferably arranged at a lower part of the bag. If the ostomy bag comprises an additional outlet opening as mentioned above, the latter may be arranged at the bottom of the bag under the outlet valve. The two openings may also be arranged in the reverse order or at the same level.

The term "closed flow path" as used herein should be interpreted as a flow path which is established between the two bags when the two valves are brought into their open positions and which is closed in the sense that the flow path is essentially sealed for preventing waste leakage during the emptying process and for preventing or at least substantially reducing odor during the emptying process.

The term "automatically" as used herein should be interpreted as the valves are brought to their open positions when the connectors are being locked and the valves are brought to their closed positions when the connectors are being unlocked without any need for two separate maneuvers for the valve operations and the locking/unlocking operations. Thus, the inventive system may be operated by a single combined locking and valve opening maneuver and a single combined unlocking and valve closing maneuver. With respect to timing, the system may be designed such that the valve opening starts when the locking is initiated and so that the valve closing starts when the unlocking is initiated. The system may also be designed such that the valve opening starts not until a while after the connectors have been locked to each other. For instance, as will be described below, if the single maneuver is a 180 degree rotational maneuver, then the locking may be initiated during the first part of the rotational maneuver, while the valve opening only starts at a later part of the rotational maneuver.

The term "connectors" is to be interpreted as elements or devices which allows the bags to be coupled together. As will be appreciated from the following description, each one of the two connectors comprised in the inventive system could be considered as a combined coupling and valve element, presenting both a coupling function and a valve function. The coupling function allows the connectors to be engaged and locked together as well as establishing a waste flow path from the ostomy bag to the collecting bag. The valve function allows the flow path to be opened and closed.

The terms "engaged", "disengaged", "locked" and "unlocked" as used in the present application should be interpreted as follows: when the two connectors are initially brought together into contact with each other with their valves closed and are properly aligned, they are in an "engaged position". When the two connectors are subsequently taken apart from each other, they are in an "disengaged position". However, an "engaged position" may be an "unlocked engaged position" or a "locked engaged position". In the unlocked engaged position, the connectors may be taken apart. In the locked engaged position, the connectors are engaged with each other and also mechanically prevented from being disengaged. The valves are open only in the locked engaged position. The valves are closed in the unlocked engaged position. The connectors can only be locked if they have first been brought into their engaged position.

The collecting bag may be a disposable bag intended for single use. The ostomy bag may be a bag intended for repeated use.

In a preferred embodiment, each one of the first connector and the second connector comprises one base member being attached to the associated bag and one moveable connector member being moveable in relation to the base member when operating the system. Each moveable connector member may comprise a moveable valve member of the valve of the associated connector.

The moveable locking element may form part of one of said moveable connector members.

In order to reduce the extent to which the first connector bulges out from the person wearing the ostomy bag, the dimensions of the first connector should preferably be reduced as much as possible, especially in the thickness direction extending out from the body. To this end, it may be preferred to design the system such that certain elements and/or functions are mainly present on the side of the second connector. Especially, a maneuver element, such as a rotational finger grip, for operating the system may preferably be arranged on the side of the second connector. Thus, the moveable part of the second connector may comprise a maneuver element. The moveable part of the second connector may preferably also comprise the moveable locking element.

In a preferred embodiment, each of the first connector and the second connector is a two-piece connector comprising one essentially stationary or fixed member and one moveable member, the latter forming a moveable valve member of the connector.

According to a preferred embodiment, the outlet valve of the ostomy bag comprises a first moveable valve member and the inlet valve of the collecting bag comprises a second moveable valve member, said first and second valve members being in operational engagement with each other when the first and second connectors are brought into engagement with each other, such that when one of the first and second valve members is moved then the other valve member is automatically also moved. The first moveable valve member and the second moveable valve members are the members being moved for opening and closing the respective valve. According to this preferred embodiment, when the connectors are in their engaged position (locked or unlocked), the valve members are in such an operational engagement with each other that if one of the valve members is moved, then the other valve member is moved along. If the valve members are rotatable valve members, the operational engagement would be implemented as a rotational lock between the valve members. An advantage of this embodiment is that the user will not have to perform separate maneuvers for each connector. If for instance the user performs a single maneuver on the second connector of the collecting bag, this single maneuver will not only open/close the second valve member but will, due to the operational engagement between the two valve members, also open/close the first valve of the ostomy bag. In addition, the single maneuver will also lock/unlock the connectors.

In a preferred embodiment, said first valve member is rotatable about a first rotational axis, said second valve member is rotatable about a second rotational axis, said first and second rotational axis being aligned with each other and forming a common rotational axis when the first and second connectors are engaged with each other, and wherein said locking element is moveable by being rotatable about said common rotational axis. According to this embodiment, a user may operate the system by a single rotational maneuver.

Other preferred or optional features are set out in the dependent claims and in the detailed description below.

According to a second aspect of the inventive concept, there is provided a connector kit for use in - or providing - an ostomy bag system as described above, said kit comprising a first connector for attachment to an ostomy bag and a second connector for attachment to a collecting bag, wherein:
said first and second connectors being connectable to each other;
said first connector being provided a waste outlet valve being moveable between a closed position and an open position;
said second connector being provided with a waste inlet valve being moveable between a closed position and an open position;
said first connector or said second connector being provided with at least one moveable locking element, said locking element being moveable between an unlocked position in which the first and second connectors are not locked to each other and a locked position in which the first and second connectors are locked to each other; and
wherein the system is arranged such that when the locking element is moved from its unlocked position to its locked position, said first and second valves are automatically brought into their open positions for defining a flow path from the first connector to the second connector, and such that when the locking element is moved from its locked position to its unlocked position, said first and second valves are automatically brought into their closed positions for closing said flow path.

According to the second aspect of the inventive concept, a user may buy or be provided with the connectors separately, in order to attach the connectors to the respective bags. In this way, different types or brands of bags may be used with the inventive connectors.

According to a third aspect of the inventive concept, there is provided a collecting bag for use in an ostomy bag system as described above, said collecting bag being provided with a second connector connectable to a first connector of an ostomy bag for receiving body waste there from, wherein:
said second connector being provided with a waste inlet valve having a second valve member being moveable between a closed position and an open position;
said second connector being provided with at least one locking element, said locking element being moveable between an unlocked position in which the first and second connectors are not locked to each other and a locked position in which the first and second connectors are locked to each other; and
said second connector being provided with a user-maneuverable element being operatively connected to the second valve member and to the locking element such that when the locking element is moved from its unlocked position to its locked position by means of said user-maneuverable element, said second valve is automatically brought into its open position for defining a flow path from the first connector to the second connector, and such that when the locking element is moved from its locked position to its unlocked position by means of said user-maneuverable element, said second valve is automatically brought into its closed positions for closing said flow path.

According to a fourth aspect of the inventive concept, there is provided a method for emptying an ostomy bag through an outlet of the ostomy bag, comprising:
connecting said ostomy bag to a collecting bag provided with an inlet, wherein the ostomy bag is provided with an initially closed outlet valve at said outlet and wherein said collecting bag is provided with an initially closed inlet valve at said inlet;
by a single locking and opening maneuver, locking the ostomy bag to the collecting bag such that they stay connected to each other during a subsequent emptying, and opening both said outlet valve and said inlet valve for providing a sealed flow path from said ostomy bag to said collecting bag;
emptying the ostomy bag into the collecting bag along said sealed flow path;
by a single unlocking and closing maneuver, unlocking said collecting bag from said ostomy bag and closing both said outlet valve and said inlet valve; and disconnecting said collecting bag from said ostomy bag.

### BRIEF DESCRIPTION OF THE DRAWINGS

In the drawings:
Fig. 1 is an exploded view of an ostomy bag system arranged on the abdomen of a person.
Fig. 2 shows the system in Fig. 1 in larger scale with connector caps in place.
Fig. 3 is a side view of the system in Fig. 2 with the connector caps removed.
Figs 4 and 5 are exploded views of the system in Fig. 1 in larger scale.
Figs 6A-D show an ostomy bag for use in the system in Figs 1-5.
Figs 7A-D show a waste collecting bag for use in the system in Figs 1-5.
Figs 8A and 8B are exploded views of a first connector aligned with a second connector.
Figs 9A and 9B show a first connector in a closed valve position.
Fig. 9C shows the first connector in a partly open valve position.
Figs 10A-C shows the first connector in an open valve position.
Fig. 11 shows a base member of the first connector.
Fig. 12A is an axial cross section of a part of the first connector in an assembled state and Fig. 12B is a perspective view of the first connector with an associated cap.
Figs 13A and 13B show a second connector in a closed valve position.
Figs 14A and 14B show the second connector in an open valve position.
Figs 15A and 15B are cross-sectional views of the second connector in its open valve position.
Fig. 16 is a perspective view of the first and second connector in an engaged position.
Fig. 17A and 17B show the first and the second connector in their engaged, locked position with their valves in open positions, establishing a sealed flow path.

### DESCRIPTION OF A PREFERRED EMBODIMENT

Referring now to the drawings in which the same reference numerals are used for designating same elements throughout the description. The figures illustrate a preferred, non-limiting embodiment of a ostomy bag system 10 according to the inventive concept.

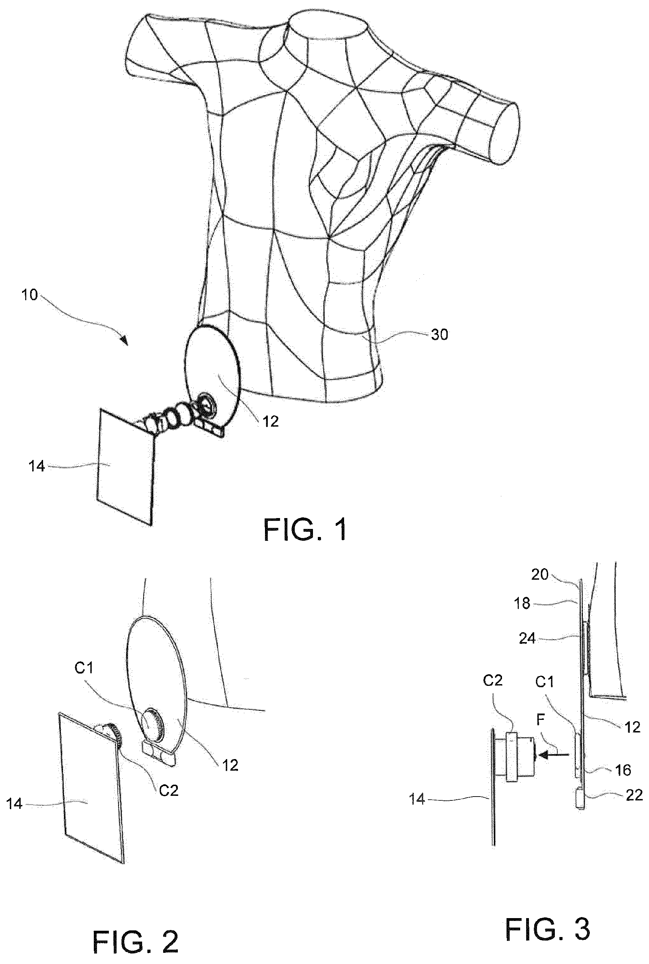

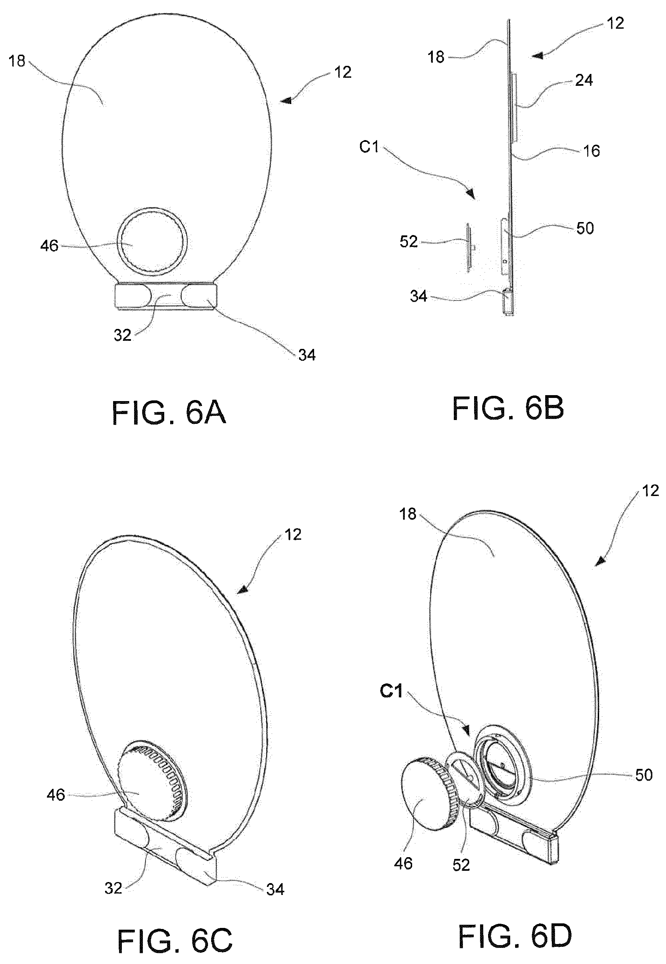

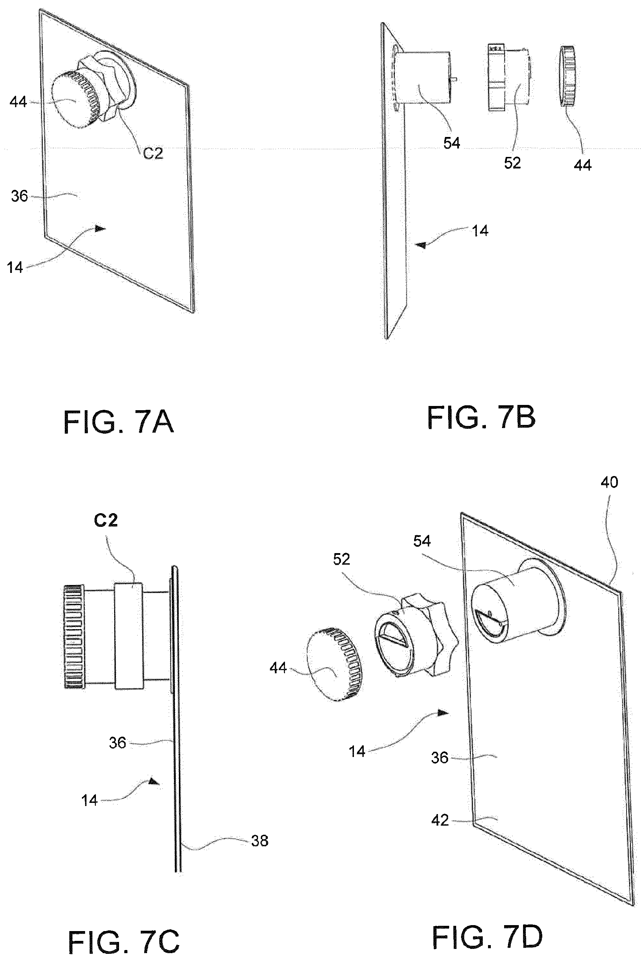

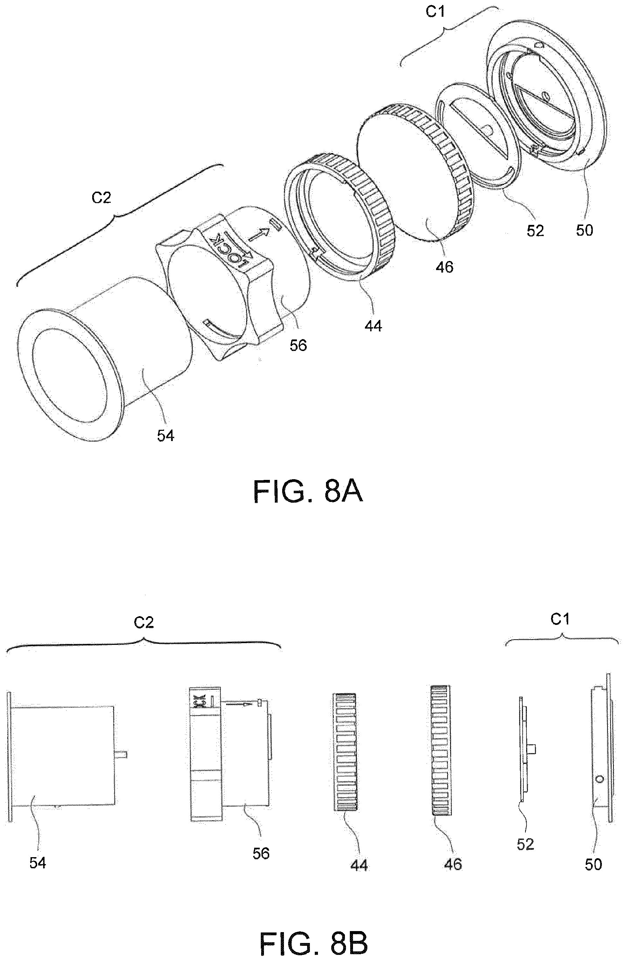

FIGS 1 to 3 illustrate an ostomy bag system 10 comprising an ostomy bag 12 and a waste collecting bag 14. The ostomy bag 12 is connectable to the waste collecting bag 14 by means of a first connector C1 of the ostomy bag 12 and a second connector C2 of the collecting bag 14. The ostomy bag 12 is illustrated in greater detail in Figs 6A-D. The waste collecting bag 14 is illustrated in greater detail in Figs 7A-D. The connectors C1 and C2 and the operation thereof is illustrated in greater details in Figs 8 to 17.

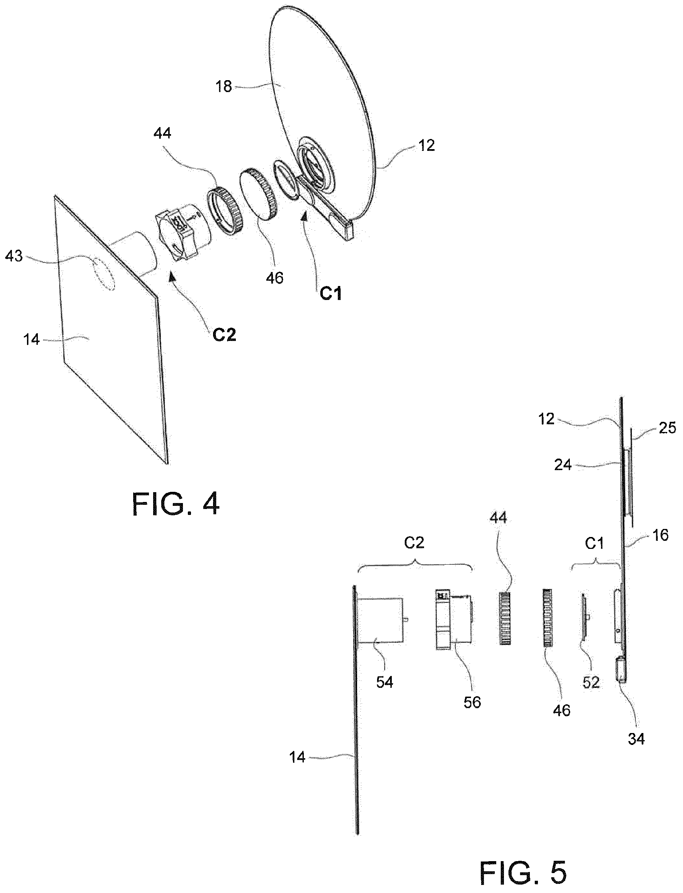

In the figures, the ostomy bag 12 is shown as being attached to the abdomen 30 of a person having a stoma (not shown). The ostomy bag 12 has a rear side 16, a front side 18, an upper end 20 and a lower end 22. As illustrated in Figs 3 and 5, the rear side 16 of the ostomy bag 12 is provided with an attachment means 24, such as a ring-shaped female connector, for attaching the ostomy bag 12 in an air-and water-tight manner to a matching connector 25, such as a ring-shaped male connector, arranged on the abdomen 30. In the attached position shown, an inlet opening (not shown) provided in the rear side 16 of the ostomy bag 12 at the attachment means 24 is located over the stoma such that the stoma can drain into the ostomy bag 12.

As known in the art, the ostomy bag 12 is provided with an unfoldable and re-foldable discharge portion or mouth 32 at the bottom end 22 of the ostomy bag 12. In the figures, the discharge portion 32 is shown in its folded, closed position. The discharge portion 32 is typically integrally formed with the rest of the ostomy bag 12. Fastening means 34, such as Velcro fasteners, securely hold the discharge portion 32 in its folded closed position. If the user desires to empty the ostomy bag 12 in a conventional manner, such as directly into a toilet as described above, the fastening means 34 are loosened such that the discharge portion 32 can be unfolded into its open discharge position. In other simpler embodiments of the inventive concept, the discharge portion 32 may be dispensed with.

The ostomy bag 12 is provided with a first connector C1 for connecting the ostomy bag 12 to a collecting bag 14. The first connector C1 is arranged over an outlet opening (not shown) in the front side 18 of the ostomy bag 12. In the present embodiment, the first connector C1 is arranged on the front side 18 of the bag 12 at the lower end 22 thereof, above the outlet portion 32. The first connector C1 is shown in an exploded view in Fig. 1, in an initial caped state in Fig. 2 and in an un-caped state in Fig. 3. The first connector C1 will be described in detail below. In some embodiment, the separate discharge portion 32 may be foldable over the first connector C1 in order to make the front of the ostomy bag less bulging and more soft.

The ostomy bag 12 may be emptied in two ways, either in the conventional manner through the discharge outlet 32 or via the first connector C1. As will be appreciated by the following description, the first alternative of using the unfoldable/re-foldable discharge outlet 32 is substantially less attractive since it involves hygienic and odor problems known in the art, but may be needed if no collection bag 14 is at hand. For both alternatives, it may be preferred to choose a bag design having a converging lower end 22, as shown in the figures, in order to make the emptying procedure as complete as possible.

In the present embodiment, the overall shape of the waste collecting bag 14 is substantially rectangular although other shapes are indeed possible. The collecting bag 14 has a front side 36, a rear side 38, an upper end 40 and a lower end 42. The collecting bag 14 is provided with a second connector C2 on its front side 36 close to the upper end 40 of the bag 14. The second connector C2 is located over an inlet opening (shown in dashed lines at 43 in Fig. 4) arranged in the front side 36 of the collecting bag 14. In the embodiment shown, the second connector C2 is arranged directly on the collecting bag 14. In other embodiments, the second connector C2 could be fluidly connected to the collecting bag via a hose or the like.

The inventive system 10 is basically used as follows. When the ostomy bag 12 needs to be emptied in a secure and sealed manner, the user takes a new, empty collection bag 14 having a lid or cap 44 (Fig. 2) removably secured over the second connector C2. The cap 44 is removed by a turning movement. A similar lid or cap 46 arranged on the first connector C1 of the ostomy bag 12 is also removed. The caps 44 and 46 form an extra security in case the sealing function of the connectors C1 and C2 for some reason should fail. Next, the second connector C2 is aligned with the first connector C1 as shown in Figs 3 to 5 and then brought into an engaged (contact) position. Next, the connectors C1 and C2 are mechanically locked to each other in a manner to be described, and a valve in each connector is opened. Waste may then be transferred from the ostomy bag 12 to the collecting bag 14 as indicated with an arrow F in Fig. 3. When the emptying procedure is completed, the valves are closed, the connectors C1 and C2 are unlocked from each other and the second connector C2 is disengaged from the first connector C1. The lids 44, 46 may be put back in position to cover the distal ends of each connector C1 and C2. The filled and closed waste collecting bag 14 may now be disposed of and the emptied ostomy bag 12 may be reused. The whole emptying procedure is completely closed and sealed and there is never any direct open access to the inside of the two bags.

The structure and operation of the first connector C1 and the second connector C2 will now be described. Figs 8A and 8B are exploded views showing the connectors C1 and C2 in an aligned arrangement, as well as the caps 44, 46. The first connector C1 comprises two members 50 and 52, and the second connector C2 also comprises two members 54 and 56. The connector members may typically be manufactured by a suitable plastic material, which typically would be a material harder than the softer material used for the bags.

The member 50 of the first connector C1 serves as a base member 50 for attaching the first connector C1 to the front side 18 of the ostomy bag 12. The other member 52 of the first connector C1 serves as a rotatable outlet valve member 52 for opening and closing an outlet valve of the first connector C1. Said outlet valve of the first connector C1 is formed in combination by the base member 50 and the outlet valve member 52. Thus, the base member 50 serves at least two functions - ostomy bag attachment and "valve housing" - but for simplicity it is termed simply "base member 50" in the following. Actually, the base member 50 has additional functions as will be described below.

The member 54 of the second connector C2 serves as a base member 54 for attaching the second connector C2 to the front side 36 of the collecting bag 14. The other member 56 of the second connector C2 serves as a rotatable inlet valve member 56 for opening and closing an inlet valve of the second connector C2. Said inlet valve of the second connector C2 is formed in combination by the base member 54 and the inlet valve member 56. Thus, the base member 54 of the second connector C2 serves at least two functions - bag attachment and "valve housing" - but for simplicity it is termed simply base member 54 in the following. Actually, the base member 54 has additional functions as will be described below.

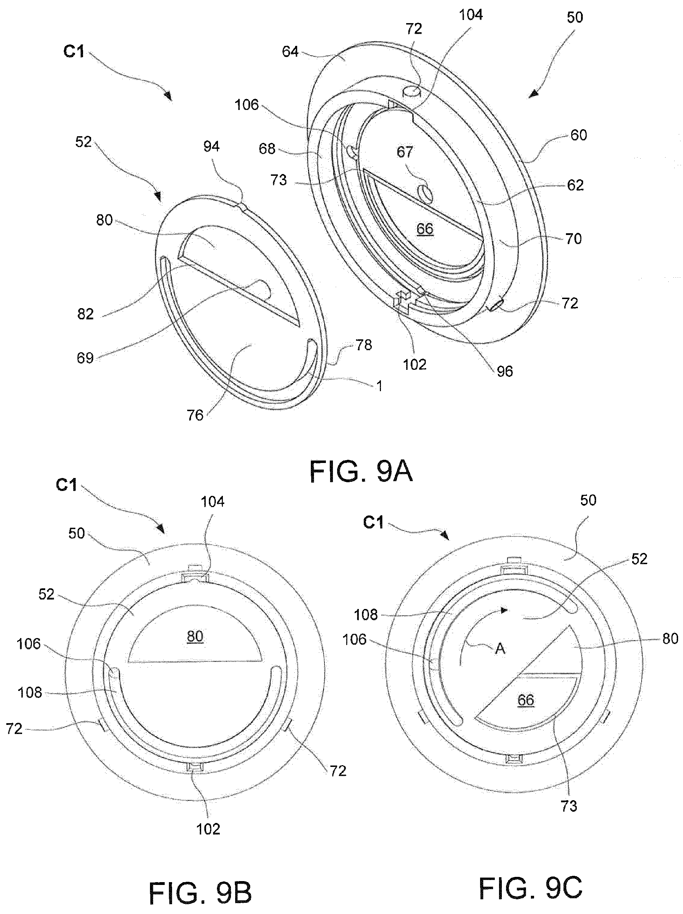

Reference is now made to Fig 9A showing an exploded view of the first connector C1 in its closed valve position, and Fig. 9B showing a corresponding front view. The base member 50 is substantially disc-shaped and comprises a disc-shaped base plate 60 and a wall-shaped connector ring 62 extending axially from a front side 64 of the base plate 60. In the embodiment shown, the base plate 60 is circular having a first diameter and the connector ring 62 is circular having a second smaller diameter. The base plate 60 and the connector ring 62 may be made in one piece. As an alternative, the base plate 60 may for comfort and body adaption reasons be manufactured in a softer more flexible material, while the connector ring 62 could be manufactured in harder material. The two parts may be manufactured in one single injection molding step although two materials are used.

The base plate 60 is sealingly attached to the ostomy bag 12. According to a first alternative, the attachment may be from the inside of the bag, such that the base plate 60 is arranged on the inside of the bag 12 and the connector ring 62 protrudes through the bag outlet opening in the front side 18 of the bag 12. The base plate 60 could then be secured by a separate locking ring (not shown) on the outside of the bag or just by adhesive. According to a second alternative, the attachment of the base plate 60 to the ostomy bag 12 may be from the outside instead, such that the rear side of the base plate 60 is secured to the outside of the ostomy bag 14 by adhesive. According to a special aspect of this second alternative, a user could himself provide an existing ostomy bag 12 with a first connector C1 by making an outlet opening by a suitable tool and then fix the base plate 60 to the front side 18 of the ostomy bag 12, or in the reverse order.

At the radially inner side of the connector ring 62, the base plate 60 is provided with a semi-circular valve opening 66, which will be referred to as the first fixed valve opening 66 in the following since it is part of the base member 50 fixed to the bag 12. In the embodiment shown, the first fixed valve opening 66 has a diameter such that the opening 66 does not extend all the way radially out to the inside 68 of the connector ring 62. In the present embodiment, the first fixed valve opening 66 extends over 180 degrees. It is also possible to implement the present invention with other angular dimensions, such as an angle being less than 180 degrees. It will also be noted that the linear base of the opening 66 is radially displaced from a central mounting opening 67 provided in the base plate 60.

Reference numeral 73 indicates a seal arranged on the front side of the base plate 60 and extending along the circumference of the first fixed valve opening 66. The seal 73, which seals against the rotatable valve member 52 in the assembled first connector C1, may be implemented in various ways. The seal 72 may be implemented as a separate sealing element attached by e.g. an adhesive to the base plate 60, either on the main surface or arranged in a recess such that the sealing element extends partly over the main surface of the base plate 60. As an alternative, the seal 72 could be manufactured in one piece with the base plate 60.

The outside 70 of the connector ring 62 is provided with a plurality of radial protrusions 72 for attaching the cap 46 by a rotational movement to the first connector C1.

The rotatable inlet valve member 52 of the first connector C1 is a circular disc-shaped element having a diameter which allows the valve member 52 to be rotatably received inside the connector ring 62 of the base member 50. The valve member 52 has a front side 76 facing the second connector C2 in use and a rear side 78 facing the base member 50 and sealingly engaging the seal 73. The valve member 52 is provided with a semi-circular valve opening 80, referred to as the first rotatable valve opening 80 in the following. In the embodiment shown, the valve opening 80 has a shape and dimensions corresponding to the shape and dimensions of the first fixed valve opening 66 of the base member 50 such that they essentially overlap in the open valve position. Reference numeral 82 indicates the surface of the valve element 52 defining the valve opening 80, corresponding to the thickness of the valve member 52.

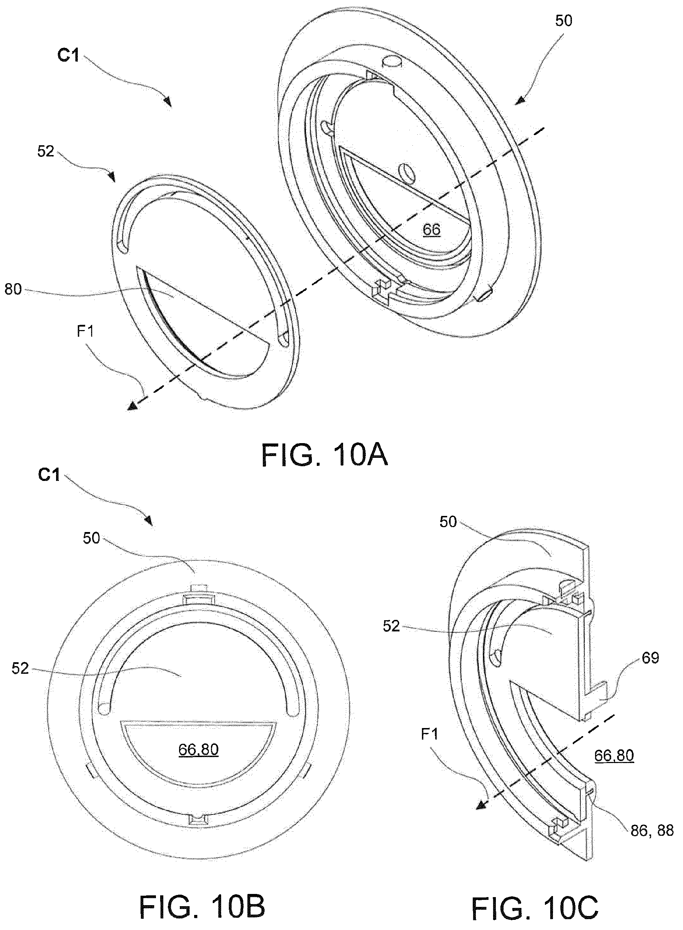

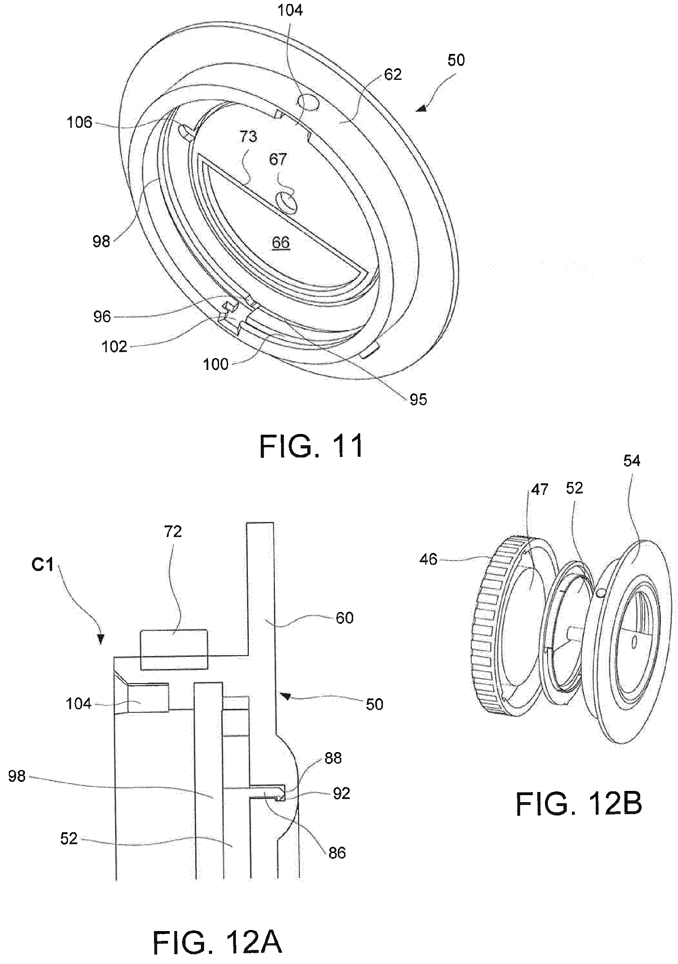

In the assembled state of the first connector C1 (Fig. 10C and Fig. 12), the rotatable valve element 52 is rotatably received inside the connector ring 62 and is prevented from disengaging from the base member 50. In the embodiment shown, this is accomplished by a central rotary attachment 67, 69 and a circumferential rotary groove-flange type attachment 86, 88. The central attachment comprises the central opening 67 in the base plate 60 and a central pin 69 extending axially from the rear side 78 of the valve member 52. The pin 69 may be rotatably secured in the central opening 67 by suitable means on the rear side of the base plate 60, such as by a click function, a separate fastening means or by melting. The circumferential groove-flange attachment 86, 88 comprises an axially directed circumferential flange 86 arranged on the rear side of the valve member 52 and a corresponding circumferential groove 88 arranged in the front side of the base plate 60. In order to reduce the overall dimensions of the base member 50 to be attached to the body, the base plate 60 is provided with a thicker portion 90 only where the groove 88 is located. The circumferential flange 86 is provided with a locking notch 92 and can be click-locked in the groove 88 as shown in Fig. 12, while still being rotatable. For increased flexibility, the flange 86 may shaped as separate, circumferentially distributed flange portions. In the preferred embodiment, the flange 86 is integrally formed with the valve member 52. It will be appreciated that the attachment of the rotatable valve member 52 to the base member 50 should be designed to allow rotation of the valve member 52 but to prevent axial removal of the valve member 52. It will also be appreciated that the attachment of the valve member 52 should be designed such that the valve member 52 can be held in a sealed engagement against the seal 73.

The base member 50 and the valve member 52 of the first connector C1 form together an outlet valve of the first connector C1. In Fig. 9A and 9B, the outlet valve is in its initial closed valve position. In this position, the lower part of the rotatable valve member 52 covers the first fixed valve opening 66 and is held in sealing engagement against the seal 73 due to the dual attachments 67/69 and 86/88 described above. In this closed valve position, the ostomy bag 12 is securely sealed. In Fig. 9C, the rotatable valve member 52 has been rotated clockwise as indicated by an arrow A into a partly open valve position. A further rotation of the valve member 52 in the direction of the arrow A will bring the first connector C1 to its fully open valve position shown in Figs 10A to 10C. In the open position, the rotary valve member 52 has been rotated 180 degrees to an end position in which the first rotatable valve opening 80 is aligned with and overlaps the first fixed valve opening 66. Thereby, an open outlet flow path F1 is established through the first connector C1.

In order to increase security against unintentional valve opening of the first connector C1, the design is preferably such as to make it hard for the user to open the outlet valve of the first connector C1 by mistake without properly using the second connector C2. The design of the present embodiment has the advantage that the rotatable valve member 50 is hard to access inside the connector ring 62. In order to further increase unintentional valve opening, the members 50, 52 are provided with positional means for holding the outlet valve in its respective closed and open valve positions. The rotatable valve member 52 is provided with small radial protrusion 94 (Fig. 9A) and the base member 50 is provided with a groove 95 (Fig. 11) which extends over 180 degrees and which at its ends has two diametrically opposed notches 96 for receiving the protrusion 94 in the rotational end positions of the rotational valve member 52 corresponding to the fully closed position and the fully open position, respectively. In this manner, the user will also receive a tactile feedback indicating when a correct rotational position has been reached.

The inventive system is a fail-proof system which allows valve opening only if the connectors C1 and C2 are locked together in their engaged position. As best shown in Fig. 11, the base member 50 is further provided with an inner locking groove 98 and an outer locking groove 100 formed in the radially inner surface of the connector ring 62. Each locking groove 98, 100 extends about 180 degrees but they are 180 degrees shifted in relation to each other. The two locking grooves 98, 100 are slightly axially displaced in relation to each other such that the inner locking groove 98 is located closer to the base plate 60 and the outer locking groove 100 is located further away from the base plate 60. The design is such that when the rotational valve member 52 is in its assembled position and attached inside the connector ring 62, then the inner locking groove 98 is so axially positioned that it is unblocked by the rotational valve member 52. The operation of these locking grooves 98, 100 will be described below. The connector ring 62 is also provided with two axially extending grooves 102, 104 formed in the radially inner surface of the connector ring 62 for providing access to the locking grooves 98, 100.

The illustrated embodiment of the inventive system may optionally be provided with a rotation-preventing function, arranged to prevent relative rotation of the two bags 12, 14. This function will be described below. For the moment, it will just be noted that the first connector C1 to this end is provided with a small recess 106 in the front side of the base plate 60 and a through-opening in the form of a semi-circular slot 108 extending about 180 degrees in the rotational valve member 52.

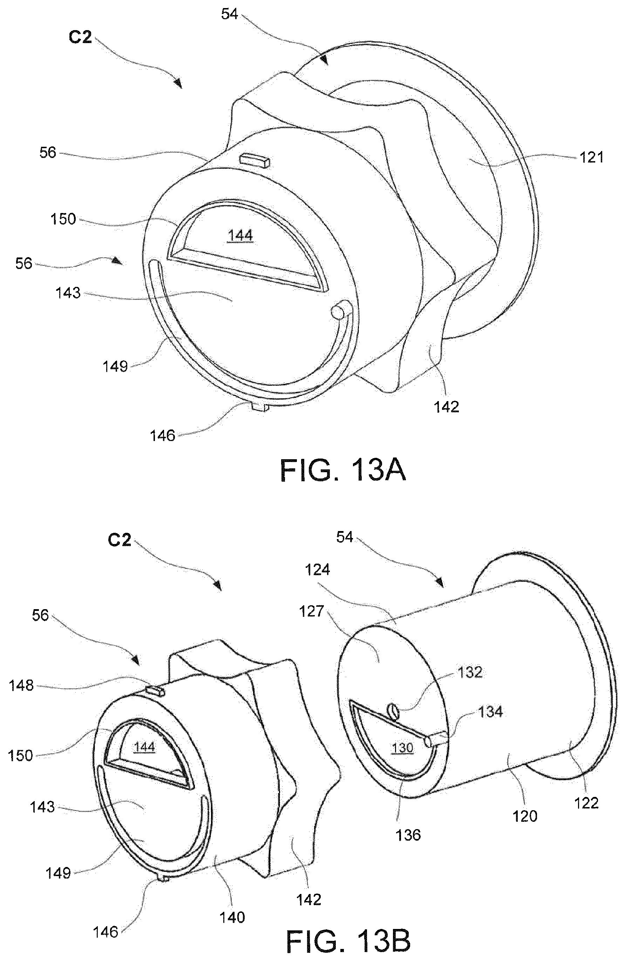

Reference is now made to Figs 13A and 13B illustrating the second connector C2 in its closed valve position. The base member 54 comprises a tubular part 120 having a rear end 122 facing the collecting bag 16 and an opposite front end 124. At the rear end 122, the base member 54 presents a circular planar attachment flange 126 allowing the base member 54 to be securely attached to the front side of the collection bag 16 in a suitable manner, such as by adhesive. At the front end 124, the base member 54 comprises a radially extending end wall 127. The end wall 127 is provided with a semi-circular through opening 130, referred to as the second fixed valve opening in the following. The opening 130 has a shape and dimensions equal to or similar to the shape and dimensions of the first fixed valve opening 66 of the first connector C1. The end wall 126 is further provided with a central attachment opening 132 and an axially extending pin 134. Similar to the first fixed valve opening 66, the second fixed valve opening 130 is provided with a seal 136 around the periphery of the opening 120. The seal 136 may be implemented in the same manner as the seal 73 of the first connector C1.

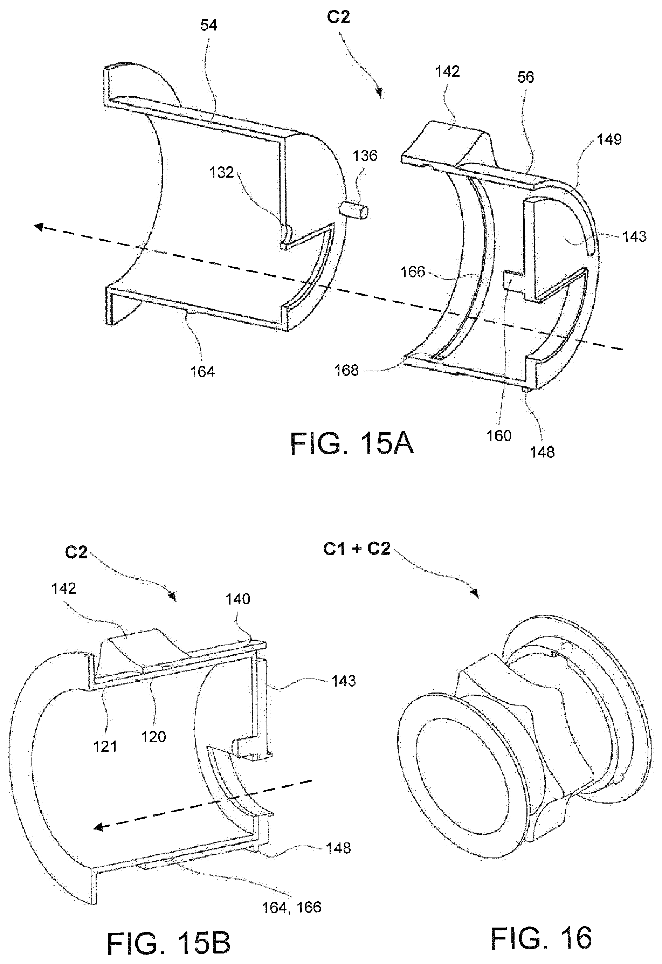

The rotatable valve member 54 of the second connector C2 comprises a tubular part 140 dimensioned to be slid over and be rotatable in relation to the tubular part 120 of the base member 54, as shown in the assembled state in Fig. 15B. The inlet valve member 54 is further provided with user maneuver means here in the form of a wave-shaped gripping element 142 arranged at a rear end of the tubular part 140. The gripping element 142 is preferably made in one piece with the tubular part 140. An end wall 143 at a front end of the tubular part 140 is provided with a semi-circular valve opening 144, referred to as the second rotatable valve opening 144 in the following. In the embodiment shown, the valve opening 144 has a shape and dimensions corresponding to the shape and dimensions of the second fixed valve opening 120 of the base member 54.

The rotatable valve member 54 is also provided with two locking elements 146 and 148, which are positioned and arranged to be received in the locking grooves 98, 100, respectively, of the base member 50 of the first connector C1 for securing the connectors C1 and C2 to each other. In the present embodiment, the locking elements 146 and 148 are in the form of two protrusions extending radially from the tubular part 140 of the rotatable valve member 56 at diametrically opposed locations. The first locking element 146 is arranged at the very distal end of the tubular part 140 to be received in the inner locking groove 98, whereas the second locking element 148 is slightly axially displaced in order to be received in the outer locking groove 100. The ends of the locking grooves 98, 100 may define the end positions of the rotational movement of the valve member 56. In a simpler embodiment, there could be one locking element only and one locking groove only. In a mechanical inversion, the locking elements could be arranged on the first connector C1 and the grooves on the second connector C2. The illustrated embodiment is preferred since it will reduce the axial dimension (thickness) of the first connector C1 of the ostomy bag 12.

One or both of the locking elements 146, 148 may be used for the attachment of the cap 44.

The rotatable valve member 54 is further provided with a through-opening in the form of a semi-circular slot 149 extending about 180 degrees in the end wall 143 of the valve member 56.

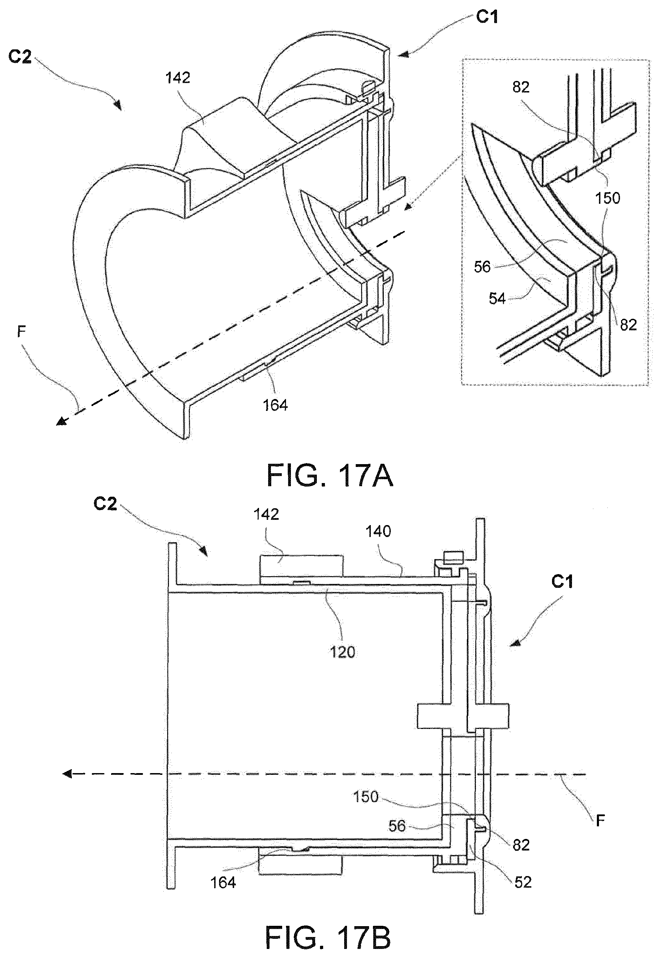

As a special feature of the rotational second valve member 56, the second rotatable valve opening 144 is provided with an axially extending rim 150, which extends along the periphery of the rotatable valve opening 144 and extends axially over a distance preferably corresponding to the thickness of the first rotational valve member 52 of the first connector C1 at the location of the surface 82 (Fig. 9A). In operation (see enlarged portion of Fig. 17A and Fig. 17B), when the first connector C1 and the second connector C2 are aligned and brought into their engaged position, the rim 150 will fit into the first rotatable valve opening 80 of the first connector C1, such that the outer side of the rim 150 engages the circumferential surface 82 of the valve opening 80. This engagement, which will be maintained both in unlocked and the locked position, has a dual function: First, the two rotatable valve members 52 and 56 will be rotationally locked to each other, such that when the valve member 56 of the second connector C2 is rotated by the user by means of the grip 142, then the rotatable valve member 52 of the first connector C1 is automatically also rotated. Second, the fact that the rim 150 extends into the valve opening 80 of the first rotatable valve member 50 has the substantial advantage of providing a closed and sealed flow path for the waste during the emptying operation. The inside of the rim 150 will of course be contaminated, but this is of less importance since the collecting bag 14 with the second connector C2 will be disposed of and any residuals on the rim 150 will be covered by the cap 46. More important, this engagement of the rim 150 into the valve opening 80 of the first connector C1 will keep the valve member 50 of the first connector C1 clean, which is obviously a substantial advantage since the ostomy bag 12 will normally be re-used a number of times. When the first rotatable valve member 50 is subsequently rotated back to its closed position and the connectors C1, C2 are disengaged, there will be no visible unclean portions of the first connector C1. Actually, the invention thereby could allow an increased number of uses of an ostomy bag 12 as compared to conventional less hygienic emptying procedures which may result in more frequent ostomy bag replacements.

Although presently considered as a less preferred alternative, it would also be possible to arrange the rim 150 on the first connector instead to be inserted into the opening 144. It would also be possible to arrange other types of rotational lock between the two rotational valve members 52, 56, but then the clean function described above would perhaps not be achieved.

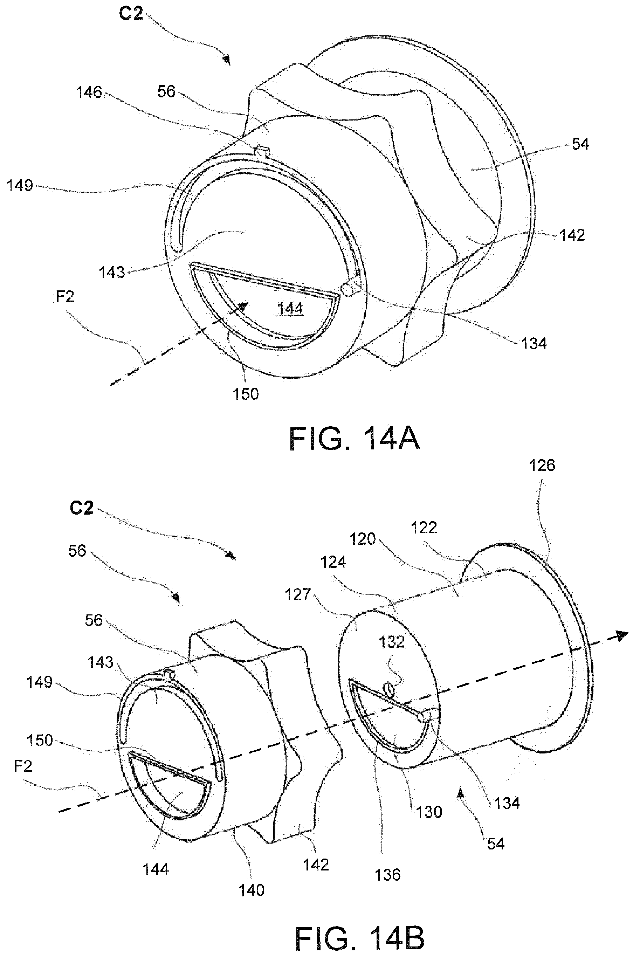

In the assembled second connector C2 (Figs 14A and 15B), the tubular part 120 of the base member 54 is partly received in the tubular part 140 of the rotatable valve member 56. A rear portion 121 of the tubular part 120 is not received in and not covered by the valve member 56. This free rear portion 121 will provide a stationary grip surface for the user during operation, a second grip surface being the rotatable grip 142. As for the first connector C1, the two members 54 and 56 are held together by two attachments, allowing the members to be clicked together during manufacturing and both allowing rotational movement. First, there is a central rotary attachment comprising a central pin 160 provided on the rotary valve member 56 and a corresponding central opening 132 in the base member 54. The pin 160 may be rotatably secured in the central opening 132 by suitable means on the rear side of the end wall 127, such as by a click function, a separate fastening means or by melting. Second, the two members 54 and 56 of the second connector C2 are held axially together by an attachment comprising a radial protrusion 164 on the outside of the tubular part 120 of the base member 54, and an associated groove 166 provided in the inside of the tubular part 140 of the rotatable valve member 56. The groove 166 extends over about 180 degrees and defines the rotary end positions of the valve member 56. Two diametrically opposed notches 168 (Fig. 15A) at either end of the groove 166 define the valve positions of the second connector C2 and provide a tactile feedback to the user when the protrusion 164 reaches the notches. As for the first connector, the protrusion 164 and the notches 168 are provided in order to prevent unintentional opening of the second connector C2.

The base member 54 and the valve member 56 of the second connector C2 form together an inlet valve of the second connector C2. In Fig. 13A and 13B, the inlet valve is in its initial closed valve position. In this position, the lower part of the rotatable valve member 56 covers the second fixed valve opening 130 and is held in sealing engagement against the seal 136 due to the dual attachments 160/132 and 164/166 described above. In this closed valve position, the collecting bag 14 is closed and securely sealed. In Figs 14A and 14B and in Figs 15A and 15B, the rotatable valve member 56 has been rotated 180 degrees into an open valve position, in which the second rotatable valve opening 130 is aligned with and overlaps the second fixed valve opening 144. Thereby, an inlet flow path F2 is established through the second connector C2.

The overall operation of the system will now be described, especially with reference to Figs. 17A and 17B showing the first connector C1 and the second connector C2 in an engaged, locked position in which both valves are open for defining a common flow path F from the ostomy bag 12 to the collecting bag 14.

Initially, the ostomy bag 12 will contain waste and the two connectors C1 and C2 would be in their closed positions.

As a first step, the caps 44, 46 are removed. The ostomy bag 12 can be maintained in attached position at the stoma as shown in Fig. 3.

Next, the two connectors C1 and C2 are axially aligned as shown in Fig. 3 such that the rotational axis of the two connectors coincide.

In order to be engaged, the two connectors C1 and C2 must also be rotationally aligned with each other. More specifically, the second connector C2 must be held such that the two locking elements 146, 148 of the second connector C2 can be received in the two axially extending grooves 102, 104 of the first connector. When held in this rotational position, the two connectors can be axially brought together into an engaged position.

In the engaged position initially obtained, the rim 150 of the valve member 150 of the second connector C2 will be received in the rotatable valve opening 80 of the valve member 52 of the first connector C1, preferably with a rather tight fit. Thereby, the rotational valve members 52 and 56 are rotationally locked, but the two valves are still closed.

In the engaged position initially obtained, there will also be established a second rotational lock, namely between the two base members 50 and 54, which rotational lock will remain during the whole operation. This rotational lock is achieved by the distal tip of the pin 134 of the second connector C2 being received in the recess 106 in the base member 50 of the first connector C1. Thereby, any unwanted relative rotation of the bags 12, 14 during the operation is prevented. Since the pin 134 extends freely through the two 180 degrees slots 108 and 149 of the two rotational valve members 52, 56, respectively, the later are free to rotate.

It will be appreciated that before any rotation of the valve members 52, 56 is initiated, the connectors C1 and C2 are only engaged, not mechanically locked to each other.

Having obtained this initial engaged, unlocked position with closed valves, the user will now turn the grip 142. The pin 134 prevents any co-rotation of the base members 50, 54. During rotation, two things will happen. First, the locking elements 144, 146 will enter the respective locking grooves 98, 100, respectively, such that the two connectors C1 and C2 will be axially locked together. Second, the valves in each connector will be gradually opened. During this valve opening, the rim 150 will be maintained received in the valve opening 80 providing a clean and closed flow path between the two connectors.

When the valve member 56 has been turned 180 degrees, the two interlocked connectors C1 and C2 will be in their fully open positions as shown in Figs 17A and 17B. This position will be noted by the user as a tactile feedback as described above. A common, closed flow path F is now established from the ostomy bag 12 to the collecting bag 14 with no risk of leakage or unwanted odor. The waste flowing along flow path F will not contaminate outer or visible parts of the valve member 52 of the first connector, such that when the emptying is completed, the visible or open parts of the first connector C1 will stay clean. Especially, the front surface of the valve member 52 will stay clean as well as the inside of the connector ring 62.

After emptying has been completed, the user will turn the grip 142 back 180 degrees in the opposite direction, whereby the valves are closed and the engagement is unlocked, such that the connectors C1 and C2 can be taken apart.

Finally, the caps 44, 46 may be put back in place.

It will thus be appreciated that the user, by a single combined locking and opening maneuver, can both lock the ostomy bag to the collecting bag such that they stay connected to each other during a subsequent emptying, as well as open both said outlet valve and said inlet valve for providing a sealed flow path from said ostomy bag to said collecting bag. After the waste in the ostomy bag has been emptied into the collecting bag along said sealed flow path, the user can, by a single combined unlocking and closing maneuver, unlock the collecting bag from said ostomy bag as well as close both said outlet valve and said inlet valve. When the unlocked position is reached, the valves have been closed.

Compared to prior art systems, the inventive system is completely fail-safe. It is impossible or at least very hard for a user to open the valve of the first connector C1 by mistake before the bags 12, 14 are sealingly locked together with a closed flow path. It is also impossible for the user to unlock the bags from each other by mistake with any of the two valves still being in an open position.

As will be seen in the figures, the locking element 146 is smaller than the locking element 148 as seen in the circumferential direction. The axial extending grooves 102, 104 are also differently sized to match the associated locking element. Thereby, the two connectors C1 and C2 can be engaged in one rotational position only in which the second connector C2 is arranged at an upper end of the collecting bag 14. In other words, the collecting bag 14 is prevented from being attached upside down.

In the embodiment shown, the valves begin to open at the same time as the locking occurs. In other embodiments it would be possible to design the valves such that valve opening does not start until the locking elements have been entered a certain distance into the locking grooves. This may be obtained by designing valve openings being less that 180 degrees. The illustrated design is presently considered to give the optimum flow path.

The system according to the invention would preferably be designed such that the dimensions of the connectors C1 and C2 are held as small as possible, but still giving an acceptable flow and acceptable locking and sealing functions. As a non-limiting example, not intended to limit the claimed scope, the following dimensions has been selected in one embodiment:
Base member 50: Axial thickness 6 mm and outer diameter 48 mm.
Valve member 52: Outer diameter 25 mm.
Valve member 56: Outer diameter 34 mm and axial length 25 mm.
Grip 142: Outer diameter 40 mm.
Caps 44, 46: Axial thickness 7 mm

The two end positions of the maneuver element 142, corresponding to the unlocked/open position and the locked/closed position, may be mechanically defined in a plurality of ways, which may be optionally used in combination. In the embodiment described, the end positions are defined by the notches 96 cooperating with the protrusion 94 and by the protrusion 164 in cooperation with the notches 168. Also, the angular movement is limited by the locking grooves 98, 100, and by the slots 108, 149.

As an optional extra security enhancing feature, the cap 46 of the first connector C1 may be designed to hold the rotational valve member 52 in its closed state when the cap 46 is mounted. With reference to Fig 12B, the inside of the cap 46 may be provided with an axially protruding engagement member 47 which will engage or lie against the front side of the valve element 52 when the cap 46 is mounted. The threads in the cap 46 may be designed such that the cap 46 is gradually displaced towards the valve member 52 when mounted on the first connector C1 such that a suitable press fit may be obtained between the member 47 and the valve member 52 to hold the latter in its closed position. In the illustrated embodiment, the member 47 is made in one piece with the cap 46 and has a diameter matching the diameter of the valve member 52. The member 47 may also be made as a separate part attached to the rest of the cap 46.

In the embodiment shown and described, the seals 73 and 136 are arranged on the non-rotating base member 50 and 54, respectively. As an alternative, seals could be arranged on the rotating valve members 52 and 56 instead, or optionally on all four members.

In a more simple embodiment, the rotational lock of the base members 50, 54 could be dispensed with. The user may then perhaps use one hand to prevent the collecting bag from rotating during opening and closing.

The valve openings 66, 80, 130, 144 may be designed with other shapes than the semi-circular shape shown. They could for instance be shaped as circular or oval holes, as circumferential slots, or even be divided into a plurality of sub-openings. In general however, it would normally be preferred to arrange a common flow path F with a single open cross section being as large as possible.

## Claims

1. An ostomy bag system (1), comprising an ostomy bag (12) for receiving bodily waste from an stoma, and a collecting bag (14) for receiving bodily waste from the ostomy bag (12) to the collecting bag, wherein:
the ostomy bag (12) is provided a first connector (C1) and the collecting bag (14) is provided with a second connector (C2), said first and second connectors (C1, C2) being connectable to each other,
said first connector (C1) is provided with a waste outlet valve being moveable between a closed position and an open position,
said second connector (C2) is provided with a waste inlet valve being moveable between a closed position and an open position,
said first connector (C1) or said second connector (C2) is provided with at least one locking element (146, 148), said locking element (146, 148) being moveable between an unlocked position in which the first and second connectors (C1, C2) are not locked to each other and a locked position in which the first and second connectors (C1, C2) are locked to each other, and
the system is arranged such that when said locking element (146, 148) is moved from its unlocked position to its locked position, said first and second valves are automatically brought into their open positions for defining a closed flow path (F) from the ostomy bag (12) to the collecting bag (14), and such that when the locking element is moved from its locked position to its unlocked position, said first and second valves are automatically brought into their closed positions for closing said flow path (F).

2. A system as claimed in claim 1, wherein the outlet valve of the ostomy bag (12) comprises a first moveable valve member (52) and the inlet valve of the collecting bag (14) comprises a second moveable valve member (56), said first and second valve members (52, 56) being in operational engagement (82, 150) with each other when the first and second connectors (C1, C2) are brought in engagement with each other, such that when one (56) of the first and second valve members is moved then the other valve member (52) is automatically also moved.

3. A system as claimed in claim 2, wherein the moveable locking element (146, 148) is operatively connected to one of said first and second valve member (52, 56).

4. A system as claimed in any of claims 1 to 3, wherein said first valve member (52) is rotatable about a first rotational axis, said second valve member (56) is rotatable about a second rotational axis, said first and second rotational axis being aligned with each other and forming a common rotational axis when the first and second connectors (C1, C2) are engaged with each other, and wherein said locking element (146, 148) is moveable by being rotatable about said common rotational axis.

5. A system as claimed in claim 4, wherein said first and second valve members (52, 56) comprise a first and a second rotatable plate-shaped element (52, 143), respectively, extending transversely to said common rotational axis.

6. A system as claimed in claim 5, wherein:
the outlet valve of the ostomy bag (12) further comprises a first non-rotatable semi-circular valve opening (66) extending transversely to the common rotational axis,
the first plate-shaped element (52) of first valve member (52) has a first semi-circular closed portion which is aligned with and closes said first non-rotatable semi-circular valve opening (66) in the closed position of the outlet valve, and a first semi-circular rotatable opening (80) which is aligned with said first non-rotatable semi-circular valve opening (66) in the open position of the outlet valve.

7. A system as claimed in claim 6, wherein the inlet valve of the collecting bag (14) further comprises a second non-rotatable semi-circular valve opening (144) extending transversely to the common rotational axis,
the second plate-shaped element (143) of the inlet valve having a second semi-circular closed portion which is aligned with and closes said second non-rotatable semi-circular valve opening (130) in the closed position of the inlet valve, and a second rotatable semi-circular opening (144) which is aligned with said second semi-circular valve opening (130) in the open position of the inlet valve.

8. A system as claimed in claim 7, wherein said second rotatable semi-circular opening (144) of the second valve member is provided with a rim (150) which extends along the circumference of the second rotatable semi-circular opening (144) and extends into and engages with said first rotatable semi-circular opening (80) of the first valve member.

9. A system as claimed in any or the preceding claims, wherein:
the moveable locking element is provided on the second connector (C1) of the collecting bag (14),
the second connector (C2) is further provided with gripping means (142) for user control of the locking element, and
said locking element, said gripping means and said second valve member are moveable as one piece (56).

10. A system as claimed in the preceding claim, wherein said gripping means, said locking element and said second valve member are integrally formed as said one piece (56).

11. A connector kit for use in or providing an ostomy bag system according to any of the preceding claims, comprising a first connector (C1) for attachment to the ostomy bag and a second connector (C2) for attachment to collecting bag, wherein:
said first and second connectors (C1, C2) being connectable to each other;
said first connector (c1) being provided a waste outlet valve being moveable between a closed position and an open position;
said second connector (C2) being provided with a waste inlet valve being moveable between a closed position and an open position;
said first connector or said second connector being provided with at least one moveable locking element (144, 146), said locking element being moveable between an unlocked position in which the first and second connectors are not locked to each other and a locked position in which the first and second connectors are locked to each other; and
wherein the system is arranged such that when the locking element (144, 146) is moved from its unlocked position to its locked position, said first and second valves are automatically brought into their open positions for defining a flow path (F) from the first connector (C1) to the second connector (C2), and such that when the locking element (144, 146) is moved from its locked position to its unlocked position, said first and second valves are automatically brought into their closed positions for closing said flow path (F).

12. A collecting bag for use in an ostomy bag system according to any of the claims 1 to 10, said collecting bag being provided with a second connector connectable to a first connector of an ostomy bag for receiving body waste there from, wherein:
said second connector being provided with a waste inlet valve having a second valve member being moveable between a closed position and an open position;
said second connector being provided with a locking element, said locking element being moveable between an unlocked position in which the first and second connectors are not locked to each other and a locked position in which the first and second connectors are locked to each other; and
said second connector being provided with a user-maneuverable element being operatively connected to the second valve member and to the locking element such that when the locking element is moved from its unlocked position to its locked position by means of said user-maneuverable element, said second valve is automatically brought into its open position for defining a flow path from the first connector to the second connector, and such that when the locking element is moved from its locked position to its unlocked position by means of said user-maneuverable element, said second valve is automatically brought into its closed positions for closing said flow path.

13. A method for emptying an ostomy bag (12) through an outlet of the ostomy bag, comprising:
connecting said ostomy bag (12) to a waste collecting bag (14) provided with an inlet, wherein the ostomy bag (12) is provided with an initially closed outlet valve at said outlet and wherein said collecting (14) bag is provided with an initially closed inlet valve at said inlet;
by a single locking and opening maneuver, locking the ostomy bag (12) to the collecting bag (14) such that they stay connected to each other during a subsequent emptying, and opening both said outlet valve and said inlet valve for providing a sealed flow path from said ostomy bag (12) to said collecting bag (14);
emptying waste from the ostomy bag (12) into the collecting bag (14) along said sealed flow path;
by a single unlocking and closing maneuver, unlocking said collecting bag (14) from said ostomy bag (12) and closing both said outlet valve and said inlet valve; and
disconnecting said collecting bag (14) from said ostomy bag (12).
